Europäisches Patentamt

European Patent Office

Office européen des brevets

(19)

(11) Numéro de publication : **0 095 986 B2**

(12) **NOUVEAU FASCICULE DE BREVET EUROPEEN**

(45) Date de publication du nouveau fascicule du brevet : **13.05.92 Bulletin 92/20**

(51) Int. Cl.⁵ : **C12N 15/81,** C12P 21/00, C12N 1/16, // C12R1/645

(21) Numéro de dépôt : **83401107.4**

(22) Date de dépôt : **01.06.83**

(54) Nouveau vecteur de clonage et d'expression, levure transformée par ce vecteur et leur application.

(30) Priorité : 02.06.82 FR 8209564
06.09.82 FR 8215114

(43) Date de publication de la demande :
07.12.83 Bulletin 83/49

(45) Mention de la délivrance du brevet :
01.06.88 Bulletin 88/22

(45) Mention de la décision concernant
l'opposition :
13.05.92 Bulletin 92/20

(84) Etats contractants désignés :
BE CH DE FR GB IT LI NL SE

(56) Documents cités :
EP-A- 0 096 430
GB-A- 2 094 341
PATENTS ABSTRACTS OF JAPAN, vol. 5, no.
98, 25 juin 1981, page (C-60)[770] & JP - A - 56
39099 (MITSUBISHI KASEI KOGYOK.K.) 14-
04-1981
JOURNAL OF BACTERIOLOGY, vol. 147, no. 1,
juillet 1981, pages 155-160, Tokyo, JP. N.
GUNGE et al.: "Intergeneric transfer ofdeoxyribonucleic acid killer plasmids, pGK11 and
pGK12, from KLUYVEROMYCES LACTIS into
SACCHAROMYCES CEREVISIAE by cell
fusion"

(56) Documents cités :
JOURNAL OF BACTERIOLOGY, vol. 148, no. 3,
décembre 1981, pages 988-990, Tokyo, JP. O.
NIWA et al.: "Curing of the killerdeoxyribonucleic acid plasmids of KLUYVEROMYCES
LACTIS"
CURRENT GENETICS, vol. 5, 1982, pages
199-203, Springer-Verlag M. WESOLOWSKI et
al.: "Killer DNA plasmids of the yeastKLUYVE-
ROMYCES LACTIS. II. Restriction Endonuclease maps"

(73) Titulaire : SOCIETE NATIONALE ELF
AQUITAINE
Tour Elf, 2, Place de la Coupole, La Défense 6
F-92400 Courbevoie (FR)

(72) Inventeur : de Louvenccourt, Laurence
65 rue E. Vaillant
F-92300 Levallois (FR)
Inventeur : Fukuhara, Hiroshi
160 Avenue du Général Leclerc
F-91190 Gif-sur-Yvette (FR)
Inventeur : Heslot, Henri
29 rue Rousselet
F-75007 Paris (FR)
Inventeur : Wesolowski, Micheline
102 Bd Kellermann
F-75013 Paris (FR)

(74) Mandataire : Warcoin, Jacques et al
Cabinet Régimbeau 26, avenue Kléber
F-75116 Paris (FR)

EP 0 095 986 B2

## Description

La présente invention concerne une levure transformée incorporant un vecteur de clonage et d'expression dans une levure, ainsi que ses applications à la synthèse de protéines.

Certaines souches de levures de l'espèce Kluyveromyces lactis contiennent un couple de plasmides linéaires dont la présence confère à la cellule le caractère "killer", cette cellule produit une toxine qui empêche la croissance d'autres cellules dites sensibles.

Parmi les cellules sensibles, il faut citer les cellules de K. lactis dépourvues de plasmides, mais également les cellules d'espèces différentes comme Saccharomyces cerevisiae.

Ces plasmides ont d'abord été décrits par Gunge et coll. (J, Bacteriol. 145, 382-390 (1981), 147, 155-166 (1981), puis indépendamment par H, Fukuhara et coll. (Current Genetics, sous presse).

Il s'agit de deux plasmides d'ADN double brin linéaires appelés $k_1$ (8,8 kb)et $k_2$ (13,4 kb).

Les études génétiques faites au Laboratoire de M. Fukuhara montrent une grande analogie avec le système "killer" de S. cerevisiae, excepté le fait que celui-ci contient deux plasmides à ARN.

Le plasmide $k_1$ est indispensable pour l'expression du caractère "killer" et de l'immunité à la toxine car la perte de ce plasmide entraîne la disparition de ces deux caractères. Il semble que le plasmide $k_2$ soit nécessaire pour la maintenance de $k_1$ dans la cellule.

La réplication de ces ADN fait intervenir plusieurs gènes chromosomiques de la levure ; l'expression de la toxine dépend également de gènes nucléaires. Il y a donc des interactions entre les deux plasmides et entre plasmides et noyau.

Or, on a découvert qu'il était possible d'utiliser le plasmide $k_1$ à titre de vecteur de clonage et d'expression.

L'utilisation de vecteurs capables de transformer une souche de Kluyveromyces, en particulier K. lactis et k. fragilis, a été décrite dans le brevet EP 0 096 430. Sur le plan pratique, l'utilisation d'un plasmide linéaire, tel que $k_1$ se heurte à des difficultés techniques importantes du fait qu'il est fort difficile à extraire de k. lactis et incapable de se multiplier dans E. Coli.

La présente invention propose donc une levure transformée incorporant un vecteur de clonage et d'expression d'un gène hétérologue dans une levure, caractérisé en ce que ledit vecteur est un plasmide circulaire qui comporte au moins :

– tout ou partie de l'ADN du plasmide $k_1$ de Kluyveromyces lactis,

– un serpent d'ADN incorporant le gène hétérologue ainsi que les séquences assurant l'expression dudit gène dans ladite levure.

L'utilisation d'un tel vecteur de clonage et d'expression est particulièrement intéressante, tout d'abord parce qu'il existe peu de vecteurs utilisables dans les levures. En outre, le plasmide $k_1$ présente plusieurs sites de restriction uniques, en particulier EcoRI, BamHI et ClaI, ce qui est particulièrement intéressant pour la construction d'hybrides recombinés.

L'utilisation d'un plasmide circulaire permet à celui-ci d'être répliqué et amplifié dans E. Coli, et donc peut être obtenu en grandes quantités avec un maniement aisé.

Enfin,, on sait que les plasmides $k_1$ sont présents à un nombre élevé de copies par cellule, ce nombre de copies pouvant atteindre 100 à 150. Dans ces conditions, on peut espérer une amplification du gène qui sera inséré dans le vecteur.

Les gènes hétérologues plus particulièrement envisagés dans le cadre de la présente invention sont des gènes codant pour la synthèse de peptides ou de protéines présentant un intérêt industriel.

Bien entendu, dans certains cas, le vecteur pourra comporter différents gènes hétérologues qui pour certains ne seront pas exprimés mais dont certaines séquences assureront l'expression d'un autre gène.

De préférence, le gène à cloner et à exprimer est inséré dans l'un des sites de restriction uniques, notamment dans le site ClaI.

Il est bien entendu possible d'insérer dans un site approprié du vecteur (c'est-à-dire en général en aval des éléments principaux assurant l'expression), si cela est intéressant, une séquence comportant de multiples sites uniques de restriction comme cela est connu dans ce domaine, afin de pouvoir commodément insérer des gènes hétérologues.

Parmi les vecteurs utilisés dans le cadre de la présente invention, il faut citer ceux incorporant à titre de gène hétérologue le gène $URA_3$ de levure, en particulier sous forme d'un segment d'ADN d'environ 1,1 kb limité par deux sites HindIII.

Des expériences ont démontré que l'intégralité du plasmide $k_1$ n'était pas indispensable pour le clonage et l'expression. Ainsi, on a pu utiliser à titre de vecteur le plasmide $k_1\delta$ qui provient d'un mutant de K. lactis NK2 "non-killer" et qui est résistant à la toxine sécrétée. Ce plasmide $k_1\delta$ présente, par rapport au plasmide $k_1$, une délétion de 2,9 kb entre les deux sites HindIII.

Les vecteurs utilisés peuvent comporter également des fragments d'ADN bactérien, en particulier des fragments d'ADN bactériens comportant une origine de replication et/ou un gène de résistance à un antibiotique, ceci notamment afin de permettre la sélection des clones dans les souches bactériennes.

Ainsi, on peut insérer un fragment de restriction de pBR322 comportant l'origine de replication et le gène de résisance à l'ampicilline.

Parmi les vecteurs utilisés certains présentent un intérêt particulier, il s'agit des plasmides circulaires tels que pL3 qui comporte à titre d'ADN du plasmide $K_1$ un fragment de restriction ClaI du plasmide $K_1$ et en outre de préférence un fragment de restriction ClaI du plasmide clone 6.

Bien entendu, les différents plasmides évoqués précédemment peuvent être préparés par la mise en oeuvre de techniques connues.

L'invention concerne également l'application des levures transformées à l'expression de la protéine codée par le gène hétérologue porté par le vecteur.

Enfin l'invention concerne un procédé de préparation d'une protéine ou d'un peptide dans lequel on cultive sur un milieu nutritif une levure transformée par un vecteur selon l'invention comportant comme gène hétérologue le gène codant pour ladite protéine ou ledit peptide.

A titre d'exemple, on décrira ci-après le clonage et l'expression du gène $URA_3$ de S. cerevisiae, en se référant en tant que de besoin aux figures annexées sur lesquelles :
– la figure 1 représente les plasmides $k_1$ et $k_1\delta$ ,
– la figure 2 représente le plasmide clone 6,

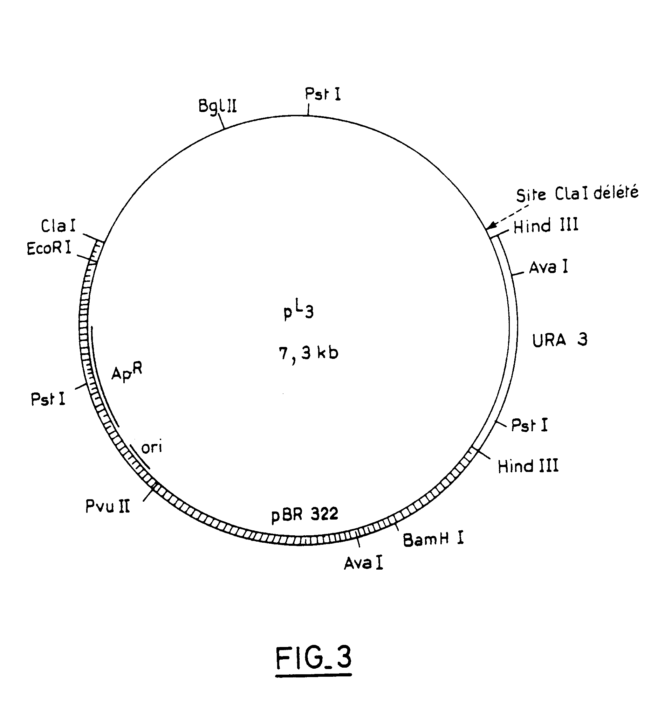

– la figure 3 représente le plasmide pL3.

## I - Construction des plasmides hybrides $k_1$-$URA_3$

Les plasmides $k_1$ et $k_1\delta$ ont déjà été décrits dans les articles mentionnés précédemment.

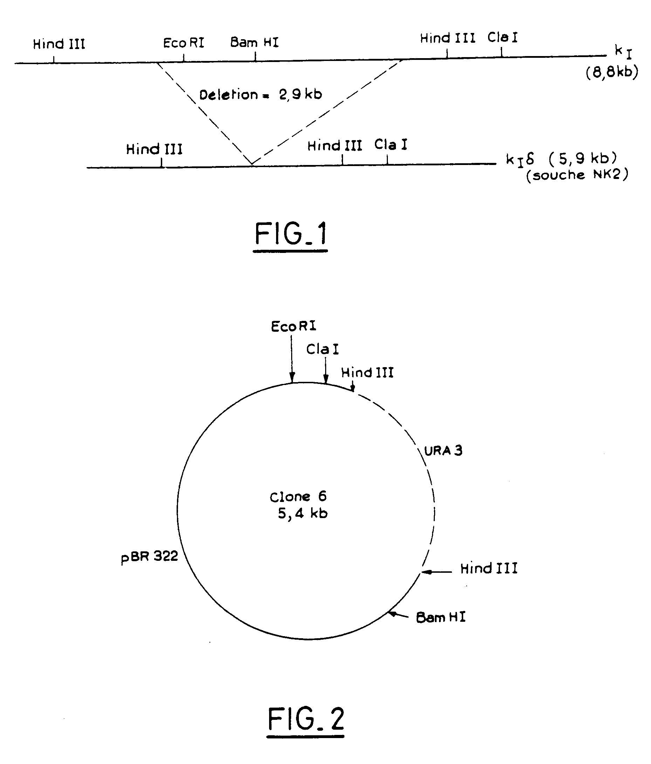

Sur la figure 1 annexée, on a représenté la carte de restriction des plasmides $k_1$ et $k_1\delta$.

Comme on peut le constater, le plasmide $k_1$ porte trois sites de restriction uniques EcoRI, BamHI et ClaI alors qu'il porte un site de restriction double HindIII.

Par contre, le plasmide $k_1\delta$, qui porte une délétion de 2,9 kb, ne comporte qu'un seul site de restriction unique ClaI mais a conservé les deux sites de restriction HindIII.

Ceci explique pourquoi, lorsque:l'on voudra cloner un gène sur le site ClaI, on pourra effectuer une restriction totale alors que, lorsque l'on voudra effectuer une restriction par HindIII, il conviendra de n'effectuer qu'une restriction partielle.

Afin d'éviter d'inactiver une région nécessaire à la réplication, le marqueur $URA_3$ a été introduit dans différents sites de $k_1$ et de $k_1\delta$.

## Premier mode de clonage : par HindIII

On effectue une restriction partielle des plasmides $k_1$ et $k_1\delta$ par HindIII.

Le gène $URA_3$ est obtenu par restriction totale par HindIII du plasmide clone 6 (décrit par Bach et coll., 1979, PNAS, 76, 386-390).

On obtient ainsi le gène $URA_3$ entier porté par un fragment d'ADN de 1,1 kb. Ce fragment est traité par la phosphatase alcaline afin d'empêcher la recircularisation de ce dernier plasmide.

Les ligations suivantes sont effectuées :
1) $k_1$ coupé par HindIII + clone 6 coupé par HindIII,
2) $K_1\delta$ coupé par HindIII + clone 6 coupé par HindIII.

## Deuxième mode de clonage : par ClaI

Le site ClaI étant unique sur les deux plasmides utilisés, $k_1\delta$ et clone 6, on peut effectuer la restriction totale des deux plasmides.

La ligation suivante est effectuée :
1) $k_1\delta$ coupé par ClaI + clone 6 coupé par ClaI.

L'ensemble de ces mélanges de ligation contiennent une certaine proportion de plasmides hybrides.

Afin de pouvoir mettre en évidence le clonage et l'expresion du gène $URA_3$, il convient de disposer d'une souche mutante de K. lactis ureaA$^-$. En outre, afin d'assurer le maintien du vecteur selon l'invention, il est intéressant que cette souche réceptrice possède également le plasmide $k_2$.

## II - Construction d'une souche mutante de K. lactis ureaA⁻ sans k₁

On est parti de la souche de <u>K. lactis</u> CBS 2360-6 uraA⁻ dépourvue d'activité OMP décase et qui a été obtenue par une mutagénèse aux UV à partir de la souche CBS 2360.

Le taux de réversion de la mutation est inférieur à $1.10^{-8}$ cellules.

Cette souche est "killer" et résistante à la toxine. Elle possède les deux plasmides $k_1$ et $k_2$, elle est notée $(k_1+, k_2+)$.

De cette souche on a éliminé le plasmide $k_1$ par un croisement avec la souche VM2 "non-killer" qui a été isolée par mutagénèse et qui présente le caractère $k_1O, k_2+$.

Le croisement suivant :

$$2360\text{-}6 \; a \; ura^- \quad X \quad VM2 \; \alpha \; lys^-$$
$$(k_1^+, \; k_2^+) \qquad\qquad (k_1^O, \; k_2^+)$$

conduit à environ 1 % de cellules diploïdes ayant perdu $k_1$ par ségrégation mitotique et qui sont devenues "non-killer".

On obtient, après sporulation de ces souches diploïdes, une souche haploïde "non-killer" ura⁻ dont on peut vérifier par extraction de l'ADN qu'elle a perdu $k_1$ et possède toujours $k_2$.

On a ainsi obtenu une souche uraA⁻ "hon-killer" ($k_1^O$, $k_2^+$) qui sera utilisée pour mettre en évidence l'expression du gène cloné sur le vecteur selon la présente invention.

## III - Transformation de K. lactis

La souche réceptrice construite précédemment est cultivée sur un milieu minimum (YNB 0,67 %, glucose 2 %) supplémenté en uracile, jusqu'à une concentration de 1 à $2.10^8$ cellules/ml.

Après deux lavages, les cellules sont remises en suspension à la concentration de $10^9$ cellules/ml dans un tampon de formation des protoplastes (KCl 0,6 M, pH 5).

On ajoute de la zymolyase 5 000 (0,5 mg/ml) et on obtient les protoplastes en 5 à 10 minutes à 34°C.

Après deux lavages, on met en présence $2.10^8$ cellules et 1 à 10 µg d'ADN des mélanges de ligation.

On ajoute 2 ml de PEG 40 %, CaCl₂ 10 mM et on laisse à la température ambiante pendant 20 minutes.

Après élimination du PEG on met en incubation 1 heure en milieu complet tamponné osmotiquement ; les protoplastes sont inclus dans de la gélose en surfusion et étalés sur un milieu minimum KCl.

Les colonies apparaissent après une semaine d'incubation à 30°C, elles sont de petites tailles et beaucoup ne poussent pas après repiquage sur milieu minimum.

L'efficacité de transformation est de 1 transformant par µg d'ADN et le taux de régénération des protoplastes est de 10 % environ.

Les résultats observés sont les suivants :

EP 0 095 986 B2

TABLEAU I

| ADN transformant | Nombre de colonies par µg ADN poussant après repiquage | Nom des transformants | "Killer" résistant | | Stabilité en milieu sélectif (%) |
|---|---|---|---|---|---|
| $k_1\delta$ et clone 6 par ClaI | 3 | L1 | "non-killer" | sensible | 0,13 |
| | | L2 | " | " | 35 |
| | | L3 | " | " | 28 |
| $k_1$ et clone 6 par HindIII | 4 | L4 | " | " | 35 |
| | | L5 | " | " | 61 |
| | | L6 | " | " | 1,5 |
| | | L7 | " | " | 0,5 |
| $k_1\delta$ et clone 6 par HindIII | 1 | L8 | " | " | 50 |

Tous les transformants figurant au tableau I sont UPA$^+$ puisqu'ils poussent sur un milieu minimum sans uracile. Ceci démontre que le gène URA$_3$ de S. cerevisiae complimente la mutation uraA$^-$ déficiente en OMP décarboxylase de K. lactis.

Les transformants ainsi obtenus ont une stabilité variable :

– cultivés en milieu minimum sans uracile, ils ségrègent entre 40 et 99 % d'ura$^-$ en 15 générations ;

– cultivés pendant le même nombre de générations sur milieu minimum + uracile, ils ségrègent entre 94 et 99 % d'ura$^-$ .

Il est toutefois possible de stabiliser le caractère URA$^+$ par des repiquages successifs sur milieu minimum.

La présence de plasmides portant k$_1$ et un ADN étranger, soit pBR322, soit URA$_3$, a été confirmée par des résultats d'hybridation sur gel ou in situ.

L'emploi de trois sondes radioactives, k$_1$, pBR325 et URA$_3$, a permis de montrer que:

L4 porte un plasmide libre de 4,4 kb visible sur gel contenant une partie de k$_1$ et le gène URA$_3$ ;

L3 porte un plasmide libre de 9 kb contenant une partie de k$_1\delta$ et une partie de l'ADN de pBR322.

On extrait l'ADN total des transformants L$_2$ et L$_3$ et on utilise cet ADN pour transformer une souche de E.coli pyr F (mutation complémentée par le gène URA3 de S. cerevisiae). On sélectionne ensuite les transformants URA$^+$ et Amp$^r$ sur les milieux appropriés et on obtient des transformants qui ont incorporé des plasmides selon l'invention.

L'extraction des plasmides issus des souches E.coli transformées par l'ADN de L$_3$ montre qu'il s'agit de plasmides conformes à la représentation de la figure annexée, ces plasmides sont dénommés p $^{\llcorner}$3. Il s'agit de plasmides circulaires ayant une taille de l'ordre de 7,3 kb qui possèdent une extrémité de k$_1\delta$ coupée par ClaI.

La figure ci-annexée représente la carte de restriction du plasmide p $^{\llcorner}$3 telle qu'elle a pu être déterminée par analyse.

Ce plasmide comprend un fragment bactérien provenant du plasmide pBR 322 et un fragment de restriction HindIII correspondant au gène URA3$^+$ de la levure S.cerevisiae, (ces deux fragments d'ADN proviennent de la restriction par ClaI du plasmide clone 6), et enfin un fragment de restriction ClaI du plasmide k$_1\delta$ de K.lactis.

Il convient de remarquer que dans p $^{\llcorner}$3 comme cela a été indiqué sur la figure l'un des sites ClaI a disparu au voisinage de l'ADN du gène URA3.

Dans ces conditions, le plasmide p $^{\llcorner}$3 comporte un grand nombre de sites de restriction uniques : ClaI, EcoRI, BamHI, ce qui le rend particulièrement apte comme vecteur de clonage de gène dans K.lactis.

En outre ce plasmide présente l'énorme avantage de pouvoir être amplifié dans E.coli et extrait facilement, ce qui en fait un plasmide particulièrement disponible.

Enfin, le plasmide p $^{\llcorner}$3 extrait de E.coli transforme les levures K. lactis ura$^-$ pour le caractère ura$^+$ avec une efficacité dix fois supérieure à celle obtenue avec les mélanges de ligation tel que cela est décrit dans le brevet principal.

Les modes opératoires ci-après sont destinés à illustrer la préparation détaillée de vecteurs selon l'invention sans pour autant la limiter.

## Les souches

Les souches de levures K. lactis utilisées sont :

– la souche CBS 2360 a (k$_1$$^+$ k$_2$$^+$) sauvage et le mutant CBS 2360 a uraA$^-$ obtenu par induction aux UV, dépourvu d'activité OMP décase ;

– la souche VM2 $\alpha$ lys$^-$ (k$_1$$^O$, k$_2$$^+$) dérivée de la souche CBS 2359.

La souche de Escherichia coli HB 101 Ap$^R$ Tet$_5$$^R$ contenant le plasmide clone 6 portant le fragment URA$_3$ de S. cerevisiae de 1,1 kb.

## Les milieux

Les levures sont cultivées en milieu minimum (glucose 2 %, "yeast nitrogen base" Difco dépourvu d'acides aminés 0,67 %, Agar Difco 2 %). Ce milieu peut être supplémenté en uracile (50 mg/l) ou lysine (40 mg/l). Le milieu YPG contient du glucose 2 %, extrait de levure 1 %, bacto peptone 1 %. Le milieu de croisement et de sporulation est le milieu ME composé d'extrait de malt : 5 % et Agar Difco 2 %. Le test "Killer" se fait sur le milieu GAL : galactose 2 %, bacto peptone 1 %, extrait de levure 1 %, KH$_2$PO$_4$ 0.05 M, Agar 2 %.

## Extraction de plasmides

L'extraction du plasmide bactérien clone 6 se fait d'après Guerry et coll. 1973 (J. Bact. 116, 1064-1066).

L'extraction et la purification des plasmides de K. lactis ont été mises au point par M. Wesolowski, P. Dumazert et H. Fukuhara, 1982, Current Genetics (sous presse).

Les cellules d'une culture de 200 ml d'YPG en fin de phase exponentielle sont lavées une fois à l'eau, pesées et suspendues tans du sorbitol 1 M (2 ml/g cellules) avec de la zymolyase 60 000 (0,5 mg/g cellules).

Après 45 minutes d'incubation à 30°C, les cellules sont centrifugées et suspendues dans une solution de NaCl 0,15 M, EDTA 0,10 M (2 ml/g cellules). On ajoute 0,5 mg/g cellules de pronase et du SDS 1 % final.

Après 1 heure d'incubation à 37°C et 1 heure à 50°C, on refroidit le tout et on ajoute de l'acétate de potassium 0,5 M final. On laisse au froid pendant 1/2 heure au moins.

Après centrifugation, le surnageant est traité à la RNase pendant 1/2 heure à 37°C. Une extraction au Sevag (chloroforme : isoamyl alcool, 24:1, vol(vol) est suivie d'une précipitation à l'éthanol (1,2 vol).

Les fibres d'ADN collectées à la pipette Pasteur sont dissoutes dans du TE (Tris 10 mM, EDTA 1 mM, pH 8). On reprécipite les fibres d'ADN avec 0,56 volume d'isopropanol redistillé et on les redissout dans du TE. Cette solution additionnée d'un colorant (bleu de bromophénol) est déposée sur un gel d'agarose à 0,6 %.

Après 18 heures de migration à 60 V, on excise les bandes révélées au bromure d'éthidium correspondant à chaque plasmide. On les électro-élue pendant une nuit à 100 mA.

L'ADN est passé ensuite sur colonne DEAE-cellulose, lavé avec NaCl 0,3 M et élué avec NaCl 2 M. L'ADN est ensuite centrifugé en gradient de CsCl, dialysé et précipité à l'éthanol.

## Restrictions, ligations

Les ADN sont restreints par les enzymes de restriction pendant 1 heure à 37°C dans le tampon correspondant à chaque enzyme. Les restrictions partielles sont réalisées avec une quantité limitante d'enzyme, pendant 5 à 10 minutes à 37°C.

La déphosphorylation est réalisée à 60°C pendant 1/2 heure avec la phosphatase bactérienne alcaline.

La ligation se fait après purification de l'ADN au phénol et précipitation à l'alcool, en présence de la ligase de T4 pendant une nuit à 10°C.

## La transformation de K. lactis

Nous nous sommes inspirés des travaux de Hinnen et coll. (1978) PNAS, 75, 1929-1933 et Gerbaud, Fournier, Blanc, Aigle, Heslot, Guérineau (1979) Gene 5, 233-253. Les cellules cultivées en milieu minimum + uracile jusqu'en phase exponentielle ($1-2.10^8$/ml) sont lavées une fois à l'eau distillée et une fois avec le tampon de protoplastisation (KCl 0,6 M pH 5).

Les cellules sont resuspendues à $10^9$ cellules/ml dans ce tampon avec 0,5 mg/ml de zymolyase 5000 et incubées un temps bref (5 à 10 minutes) à 34°C.

On obtient plus de 90 % de protoplastes.

On lave deux fois les protoplastes avec un tampon Tris HCl, pH 7,5, KCl 0,6 M, $CaCl_2$ 10 mM en centrifugeant à 1800 g, 5 minutes et en resuspendant les cellules délicatement ; elles sont concentrées à $10^9$ cellules/ml dans ce dernier tampon. On ajoute 1 à 10 µg de mélange de ligation à 0,2 ml de cellules ($2.10^8$ cellules) et on mélange bien.

Après 15 minutes d'incubation à la température ambiante, on ajoute 2 ml de PEG 4000 à 30 % (en poids/volume) et on laisse 15 minutes à température ambiante.

Après centrifugation à 1800 g, 6 minutes, on resuspend dans 2 ml de KCl 0,6 M, glucose 6 g/l, Bacto peptone 6 g/l, extrait de levure 4 g/l, et on agite doucement à 30°C pendant 1 heure.

Les cellules sont ensuite centrifugées et resuspendues dans 0,2 ml de tampon de protoplastisation. On ajoute 5 ml de gélose en surfusion à 46°C (KCl 0,6 M, glucose 2 %, YNB "sans aminoacides" Difco 0,67 %, Agar Difco qualité "purified" 30 g/l). Le tout est coulé sur une boite préchauffée à 55°C contenant un milieu de même composition que la gélose ci-dessus mais avec un Agar Difco normal.

## L'hybridation

1) Préparation des filtres d'hybridation sur colonie (in situ), (Grünstein M. et Hogness D.S. (1975) PNAS 72, 3961-3965).

Les colonies poussant sur W sont répliquées sur un filtre Schleicher et Schüll BA posé sur une boîte de W et mises à incuber à 30°C pendant 2 jours.

La protoplastisation se fait en 1/2 heure à 30°C avec de la zymolyase 60 000 (I mg/ml), en KCl 0,6 M. Après séchage sur papier Whatman, les filtres sont transférés successivement sur papier Whatman imbibé de :

– NaOH 0,5 N (5 minutes)
– Tris HCl pH 7,5 1 M (2 x 2 minutes)
– NaCl 1,5 M, Tris HCl 0,5 M pH 7,5 (2 x 5 minutes).
Les filtres sont séchés sous vide à 80°C pendant 3 heures.

2) L'hybridation sur gel est réalisée suivant Southern E.M. (1975) J. mol. Biol. 98, 503-517.

3) Les filtres sont traités à 65°C dans différents bains :

. la préhybridation se fait pendant 30 minutes dans du 3 x SSC (20 x SSC = 3 M NaCl 0,3 M Na citrate) puis pendant 3 heures dans le tampon 3 x SSC, 10 x Denhardt (Denhardt = Ficoll 400 0,2 %, serum albumine bovine 0,2 %, polyvinylpyrrolidone 0,2 %), enfin, pendant 2 heures dans le même tampon additionné de 50 µg/ml d'ADN compétiteur, 0,1 % SDS, 0,1 M $NaH_2PO_4$ pH 5,6, 10 % dextrane sulfate.

. Pour l'hybridation des filtres avec la sonde radioactive marquée au $^{32}P$ par nick-translation, on ajoute la sonde dénaturée au dernier bain de préhybridation et on laisse incuber une nuit à 65°C. La nick-translation est décrite par Cameron et coll. 1979, Cell, 16, 739-751, ainsi que par les données du Centre de Radio-chimie d'Amersham.

. Les 6 premiers lavages des filtres se font dans du 3 x SSC 10 x Denhardt, 0,1 % SDS (avec 50 µg/ml d'ADN compétiteur lors du premier lavage seulement) puis les deux derniers lavages dans du 1 x SSC. Les filtres sont séchés et révélés par des films Kodak.

**Revendications**

1. levure transformée incorporant un vecteur de clonage et d'expression d'un gène hétérologue dans une levure, caractérisée en ce que ledit vecteur est un plasmide circulaire qui comporte au moins :
– tout ou partie de l'ADN du plasmide $k_1$ de Kluyveromyces lactis,
– un segment d'ADN incorporant le gène hétérologue ainsi que les séquences assurant l'expression dudit gène dans ladite levure.

2. levure selon la revendication 1, caractérisée en ce que le vecteur comporte également des fragments d'ADN bactérien.

3. levure selon la revendication 2, caractérisée en ce que les fragments d'ADN bactérien comportent une origine de réplication.

4. levure selon la revendication 3, caractérisée en ce que l'origine de réplication est l'origine de réplication de pBR322.

5. levure selon l'une des revendications 1 à 4, caractérisée en ce que le segment d'ADN du gène hétérologue est un segment d'ADN du gène $URA_3$ de Saccharomyces cerevisiae.

6. levure selon l'une des revendications 1 à 4, caractérisée en ce que le vecteur ne comporte qu'une partie de l'ADN du plasmide $k_1$ correspondant à l'ADN du plasmide $k_1\delta$.

7. levure selon l'une des revendications 1 à 3, caractérisée en ce que le vecteur est un plasmide circulaire comportant à titre d'ADN du plasmide $k_1$ de K. lactis un fragment de restriction ClaI du plasmide $k_1\delta$.

8. levure selon la revendication 7, caractérisée en ce que le vecteur comporte un fragment de restriction ClaI du plasmide clone 6 comportant un fragment de pBR322 et le gène $URA_3$.

9. levure selon l'une des revendications 7 et 8, caractérisée en ce que le vecteur est le plasmide $p^L3$ comportant un fragment de pBR322, le gène $URA_3$+ et un fragment ClaI du plasmide $k_1\delta$.

10. levure selon la revendication 9, caractérisée en ce qu'il s'agit d'une souche de Kluyveromyces.

11. levure selon la revendication 10, caractérisée en ce qu'il s'agit d'une souche de Kluyveromyces lactis.

12. Souche de Kluyveromyces lactis selon l'une des revendications 10 et 11, caractérisée en ce qu'il s'agit d'une souche de K. lactis ($k_1$o, $k_2$+).

13. Souche de Kluyveromyces lactis transformée par un plasmide selon la revendication 1, caractérisée en ce que ledit plasmide vecteur comporte :
– un fragment de restriction ClaI du plasmide $k_1\delta$,
– un fragment de restriction ClaI du plasmide clone 6 portant le gène $URA_3$ et un fragment de pBR322, ladite souche de K. lactis étant uraA$^-$, $k_1$o, $k_2$-.

14. Application d'une levure selon l'une des revendications 1 à 11 à la préparation d'une protéine codée par le gène hétérologue porté par le vecteur.

**Patentansprüche**

1. Transformierte Hefe, die einen Vektor zur Klonierung und Expression eines heterologen Gens in einer Hefe einschließt, dadurch gekennzeichnet, daß besagter Vektor ein zirkuläres Plasmid ist das wenigstens:
– ganz oder teilweise die DNA des Plasmids $k_1$ von <u>Kluyveromyces lactis,</u>
– ein DNA-Segment, das das heterologe Gen ebenso wie die Sequenzen, die die Expression besagten Gens in besagter Hefe sicherstellen, einschließt, umfaßt.

2. Hefe nach Anspruch 1, dadurch gekennzeichnet, daß der Vektor auch bakterielle DNA-Fragmente umfaßt.

3. Hefe nach Anspruch 2, dadurch gekennzeichnet, daß die bakteriellen DNA-Fragmente einen Replikationsursprung umfassen.

4. Hefe nach Anspruch 3, dadurch gekennzeichnet, daß der Replikationsursprung der Replikationsursprung von pBR 322 ist.

5. Hefe nach einem der Ansprüche 1 bis 4, dadurch gekennzeichnet, daß das DNA-Segment des heterologen Gens ein DNA-Segment des Gens $URA_3$ von <u>Saccharomyces cerevisiae</u> ist.

6. Hefe nach einem der Ansprüche 1 bis 4, dadurch gekennzeichnet, daß der Vektor nur einen Teil der DNA des Plasmids $k_1$ umfaßt, welcher der DNA des Plasmids $k_1\delta$ entspricht.

7. Hefe nach einem der Ansprüche 1 bis 3, dadurch gekennzeichnet, daß der Vektor ein zirkuläres Plasmid ist, das als DNA des Plasmids $k_1$ von <u>K. lactis</u> ein Restriktionsfragment ClaI des Plasmids $k_1\delta$ umfaßt.

8. Hefe nach Anspruch 7, dadurch gekennzeichnet, daß der Vektor ein Restriktionsfragment ClaI des Plasmidklons 6 umfaßt, einschließlich eines Fragments von pBR 322 und des Gens $URA_3$.

9. Hefe nach einem der Ansprüche 7 und 8, dadurch gekennzeichnet, daß der Vektor das Plasmid $p^L3$ ist, das ein Fragment von pBR 322, das Gen $URA_3+$ und ein Fragment ClaI des Plasmids $k_1\delta$ umfaßt.

10. Hefe nach Anspruch 9, dadurch gekennzeichnet, daß es sich um einen Stamm von <u>Kluyveromyces</u> handelt.

11. Hefe nach Anspruch 10, dadurch gekennzeichnet, daß es sich um einen Stamm von <u>Kluyveromyces lactis</u> handelt.

12. Stamm von <u>Kluyveromyces lactis</u> nach einem der Ansprüche 10 und 11, dadurch gekennzeichnet, daß es sich um einen Stamm von <u>K. lactis</u> ($k_1o$, $k_2+$) handelt.

13. Stamm von <u>Kluyveromyces lactis</u>, transformiert durch ein Plasmid nach Anspruch 1, dadurch gekennzeichnet, daß besagter Plasmidvektor:
– ein Restriktionsfragment ClaI des Plasmids $k_1\delta$ ,
– ein Restriktionsfragment ClaI des Plasmidklons 6, einschließlich des Gens $URA_3$ und eines Fragments von pBR 322 umfaßt,
wobei besagter Stamm von <u>K. lactis</u> uraA$^-$ , $k_1o$, $k_2$- ist.

14. Verwendung einer Hefe nach einem der Ansprühe 1 bis 11 zur Herstellung eines Proteins, das von dem heterologen Gen kodiert wird, das der Vektor trägt.

**Claims**

1. Transformed yeast incorporating a cloning and expression vector of a heterologous gene in a yeast, characterized in that said vector is a circular plasmid containing at least :
– all or part of the DNA of plasmid $k_1$ of <u>Kluyveromyces lactis,</u>
– a DNA segment incorporating the heterologous gene and sequences which ensure expression of the said gene in the said yeast.

2. Yeast according to claim 1, characterized in that the vector contains also fragments of bacterial DNA.

3. Yeast according to claim 2, characterized in that the fragments of bacterial DNA contain an origin of replication.

4. Yeast according to claim 3, characterized in that the origin of replication is the origin of replication of pBR322.

5. Yeast according to one of claims 1 to 4, characterized in that the DNA segment of the heterologous gene is a DNA segment of the $URA_3$ gene of <u>Saccharomyces cerevisiae.</u>

6. Yeast according to one of claims 1 to 4, characterized in that the vector contains only one part of the DNA of plasmid $k_1$ corresponding to the DNA of plasmid $k_1\delta$.

7. Yeast according to one of claims 1 to 3, characterized in that the vector is a circular plasmid containing as the DNA of plasmid $k_1$ of <u>K. lactis</u> a Cla I restriction fragment of plasmid $k_1\delta$.

8. Yeast according to claim 7, characterized in that the vector contains a Cla I restriction fragment of the clone 6 plasmid containing a fragment of pBR322 and the $URA_3$ gene.

9. Yeast according to one of claims 7 and 8, characterized in that the vector is the plasmid $p^L3$ containing a fragment of pBR 322, the $URA_3^+$ gene and a Cla I fragment of the plasmid $k_1\delta$.

10. Yeast according to claim 9, which is a strain of <u>Kluyveromyces</u>.

11. Yeast according to claim 10, which is a strain of <u>Kluyveromyces lactis</u>.

12. Strain of <u>Kluyveromyces lactis</u> according to one of claims 10 and 11, which is a strain of <u>K. lactis</u> ($k_1^O$, $k_2^+$).

13. Strain of <u>Kluyveromyces lactis</u> transformed by a plasmid according to claim 1, in which said plasmid vector contains,

– a Cla I restriction fragment of the plasmid $k_1\delta$,

– a Cla I restriction fragment of the clone 6 plasmid carring the $URA_3$ gene and a fragment of pBR 322, said strain of <u>K. lactis</u> being ura $A^-$, $k_1^O$, $k_2^+$.

14. Use of a yeast according to one of claims 1 to 11 in the preparation of a protein encoded by the heterologous gene carried by the vector.

## FIG.1

## FIG.2

FIG.3